# FASCICULE DE BREVET EUROPEEN

(11) **EP 1 227 814 B1**
(45) Date de publication et mention de la délivrance du brevet: **05.01.2005**
(21) Numéro de dépôt: 00971476.7
(22) Date de dépôt: 24.10.2000
(51) Int. Cl.: A61K 31/57, A61K 31/565, A61P 5/30

(54) **COMPOSITION HORMONALE A BASE DE ACETATE DE NOMEGESTROL ET D'UN ESTROGENE ET SON UTILISATION**
NOMEGESTROL ACETAT UND ESTROGEN ENTHALTENDE ZUSAMMENSETZUNG UND IHRE VERWENDUNG
HORMONAL COMPOSITION BASED ON NOMEGESTROL ACETATE AND AN OESTROGEN AND USE THEREOF

(30) Priorité: 25.10.1999 WO PCT/FR99/02588
(43) Date de publication de la demande: 07.08.2002
(73) Titulaire: LABORATOIRE THERAMEX, 98000 Monaco (MC)
(72) Inventeur: PARIS, Jacques, Le Clos de Cimiez, F-06100 Nice (FR); THOMAS, Jean-Louis, F-94220 Charenton-le-Pont (FR)
(74) Mandataire: Gillard, Marie-Louise
(86) Numéro de dépôt international: PCT/FR2000/002939
(87) Numéro de publication internationale: WO 2001/030357

(56) Documents cités:
- EP-A- 0 136 011
- FR-A- 2 754 179
- US-A- 5 208 225

## Description

La présente invention se rapporte au domaine de la chimie thérapeutique et plus particulièrement au domaine de la technique pharmaceutique.

Elle a plus précisément pour objet l'utilisation de compositions pharmaceutiques formées d'une association estroprogestative en vue de la correction des carences estrogéniques chez la femme, quelle qu'en soit l'origine, et plus spécialement chez la femme ménopausée.
Elle a en particulier pour objet l'utilisation d'une association estroprogestative constituée par des unités de prise renfermant une combinaison d'un progestatif et d'un estrogène, les deux composants étant présents simultanément dans chaque prise médicamenteuse.
Elle a spécifiquement pour objet l'utilisation de compositions pharmaceutiques destinées au traitement hormonal substitutif de la ménopause, renfermant comme principe actif un agent progestatif choisi parmi le nornegestrol et ses esters et un agent estrogène choisi parmi l'estradiol et ses esters et les estrogènes conjugués équins.

Cette association est destinée à être administrée par voie orale, de façon continue ou discontinue.

Comme on le sait, l'espérance de vie de la femme est passée en moins d'un siècle de 50 à 80 ans, tandis que l'âge moyen de survenue de la ménopause est resté inchangé. Ainsi, les femmes passent près du tiers de leur vie en état de carence estrogénique, ce qui est à l'origine de l'augmentation du risque d'ostéoporose et de maladies cardiovasculaires. Le traitement substitutif de la ménopause est donc devenu très courant. Il est administré soit par voie orale soit, au moins pour sa composante estrogénique, par voie percutanée. Néanmoins, l'observance paraît meilleure lorsque le traitement est administré per os (ETTINGER et al, 1998).

Le traitement substitutif séquentiel de la ménopause guérit la symptomatologie climatérique. Il prévient l'ostéoporose et la survenue des maladies cardiovasculaires. Il crée des cycles artificiels qui sont suivis par une hémorragie de privation. Ce schéma thérapeutique convient tout particulièrement aux femmes dont la ménopause est récente mais il n'est pas toujours bien accepté à long terme, ce qui explique en partie la médiocre observance du traitement (DRAPIER FAURE E. ; Gynécologie, 1992, 43: 271-280 ).

Pour pallier cet inconvénient, on a mis au point des associations combinées où les deux composants sont pris simultanément, de façon continue ou discontinue, le progestatif ayant pour effet de s'opposer en permanence à l'action proliférative de l'estrogène sur l'endomètre, en provoquant une atrophie de l'endomètre et, par voie de conséquence, l'absence d'hémorragie de privation (HARGROVE J.T., MAXSON W.S., WENTZ A.C., BURNETT L.S, Obstet Gynecol, 1989, 73 : 606-612 ). En effet, dans ces conditions, l'atrophie endométriale est forte (WOLFE et PLUNKETT, 1994; PIEGSA et al, 1997; AFFINITO et al, 1998), il n'y a pas d' hyperplasie endométriale (STADBERG et al, 1996) et la fréquence des saignements est faible et diminue avec le temps (PIEGSA et al, 1997; CARRANZA-LIRA, 1998; ETTINGER et al, 1998). Avec ce type de traitement, l'observance est, d'une façon générale, bonne (EIKEN et KULTHOFF, 1995; DOREN et al, 1996), et meilleure qu'avec un traitement séquentiel (EIKEN et al, 1996). La qualité de vie paraît, elle aussi, améliorée (ULRICH et al,1997). On sait aussi que ce type de traitement protège la masse osseuse (EIKEN et al, 1996; EIKEN et al, 1997; HART et al, 1998; RECKER et al, 1999).

Ce schéma "sans règles " convient plus particulièrement aux femmes dont la ménopause est déjà ancienne. Il peut être prescrit en relais des associations séquentielles afin d'améliorer l'observance au long cours du traitement hormonal substitutif de la ménopause.

Lors des traitements séquentiels, la dose de progestatif choisie est celle qui conduit à long terme à moins de 1 % d'hyperplasie endométriale lorsque le progestatif est administré en discontinu, plus de 10 jours par cycle, chez les femmes ménopausées sous estrogénothérapie substitutive (WHITEHEAD et coll., J. Reprod. Med, 1982, 27 : 539-548, PATERSON et coll., Br Med J, 1980, 22 March : 822-824).
La dose de progestatif à utiliser dans un traitement substitutif combiné est en général déduite de celle qui est habituellement prescrite dans les schémas séquentiels. C'est l'exemple de la progestérone micronisée, de la didrogestérone (FOX H., BAAK J., VAN DE WEIJER P., AL-AZZAWI E., PATERSON M., JOHNSON A., MICHELL G., BARLOW D., FRANCIS R., 7 th International Congress on the Menopause, Stockholm, 20-24 Juin 1993, abstr 119) et de l'acétate de médroxyprogestérone (BOCANERA R., BEN J., COFONE M., GUINLE I., MAILAND D., SOSA M., POUDES G., ROBERTI A., BISO T., EZPELETA D., PUCHE R., TOZZINI R., 7 th International Congress of the Menopause, Stockholm, 20-24 Juin 1993, abstr 40) qui ont été utilisées respectivement à la posologie de 100, 10 et 5 mg/jour, avec des résultats encourageants sur le plan clinique et endométrial.
Le traitement combiné est le plus souvent utilisé de façon continue, c'est-à-dire sans interruption. Certains sont cependant partisans de l'utiliser de façon intermittente, par exemple 25 jours par mois (BIRKAUSER M et al ; Substitution hormonale : une indication bien posée et des schémas de traitement individuels sont déterminants pour le succès du traitement, Méd et Hyg, 1995, 53 : 1770-1773). L'interruption thérapeutique a pour but de faire disparaître l'inhibition exercée par le progestatif sur la synthèse des récepteurs de l'estradiol et de la progestérone et éviter ainsi la baisse de réceptivité des tissus hormone-dépendants.

Le progestatif utilisé selon la présente invention est le nomegestrol ou un de ses esters, notamment l'acétate de nomégestrol. L'acétate de nomegestrol est un progestatif puissant, actif par voie orale et qui a un profil pharmacologique original :
- à l'inverse des dérivés de 19-nortestosterone, l'acétate de nomegestrol ne manifeste aucune activité androgénique et estrogénique résiduelle;
- comme les dérivés de la 17alpha-hydroxyprogesterone, il présente un profil pharmacologique pur, mais à l'inverse de ceux-ci, il a un puissant effet antigonadotrope.

Il appartient à la catégorie des progestatifs qualifiés d'hybrides (OETTEL et al, 1999) qui ne sont pas porteurs d'effets métaboliques délétères du fait de l'absence de fonction 17alpha-ethinyl et qui combinent les avantages des dérivés de progestérone avec ceux des dérivés plus modernes de la 19-nortestosterone.
Son utilisation en administration séquentielle lors de la ménopause à la dose de 5 mg/jour. 12 jours par cycle, en association avec différents types d'estrogènes, permet de prévenir l'hyperplasie endométriale comme l'a montré une étude multicentrique sur 150 femmes durant 1 an (THOMAS J.L, BERNARD A.M., DENIS C, 7 th International Congress on the Menopause, Stockolm, 20-24 Juin 1993, abstr 372 ).
L'absence d'hyperplasie a été confirmée dans une étude où l'acétate de nomegestrol a été administré à la même dose, 14 jours par cycle, chez des femmes traitées par l'estradiol percutané (BERNARD A.M. et al. Comparative évaluation of two percutaneous estradiol gels in combination with nomegestrol acetate in hormone replacement therapy. XIV World Congress of Gynecology and Obstetrics, FIGO, Montréal, 24-30 September 1994). Cette utilisation, objet du brevet français 2.737.411 au nom de la Société Demanderesse revendique une fourchette de doses entre 1,5 et 6 mg, de préférence entre 2,5 et 5 mg.

L'estrogène utilisé est l'estradiol, fibre ou estérifié, et notamment le valérate d'estradiol, ou des estrogènes conjugués équins, qui se présentent selon une formulation active par voie orale. Il a été montré qu'une dose d'estradiol comprise entre 1 et 2 mg/jour permettait de combattre l'hypoestrogénie présente chez les femmes ménopausées.

L'acétate de nomegestrol et l'estradiol libre ou estérifié, ou les estrogènes conjugués équins sont administrés sous une des formes permettant l'administration par voie orale : gélules, capsules, pilules, sachets de poudre, comprimés, comprimés enrobés, dragées etc.
La présente invention est caractérisée par l'utilisation d'une association estroprogestative, active par voie orale, administrée d'une manière combinée. La présente invention a également pour objet l'utilisation des compositions selon l'invention dans la correction des carences estrogéniques, dans la prévention de l'ostéoporose et des maladies cardio-vasculaires chez la femme ménopausée.

La présente invention se définit également par :

### a) le fait que l'association estroprogestative est différente de celles décrites précédemment pour le même type d'indications.

Certains brevets revendiquent l'utilisation de combinaisons estro-progestatives en continu pour le traitement substitutif de la ménopause. C'est par exemple le cas des brevets US 5108995 ou EP 309263. Cependant, de façon évidente, ces brevets revendiquent des traitements multiséquentiels avec des changements de doses des principes actifs. C'est aussi le cas des brevets déposés aux USA (US 4820831 A) et en Europe (EP 136 011) au nom de PLUNKETT. Ces brevets revendiquent l'usage de nombreux estrogènes et de nombreux progestatifs dans le traitement substitutif de la ménopause. Il apparaît cependant que ces revendications ne couvrent pas l'usage de tous les progestatifs d'une part pour des raisons scientifiques et d'autre part pour des raisons scientifiques et juridiques liées à la formulation des revendications de ces deux brevets :
1) L' utilisation de nombreux progestatifs est basée sur des équivalences avec l'un d'entre eux, en l'occurrence le levonorgestrel. Cette approche n'apparait pas recevable car les différents progestatifs se caractérisent par des profils pharmacologiques très différents et il n'est pas possible de déduire les doses à utiliser par un système d'équivalence simple et unique. Cette impossibilité devient évidente si on considère les fourchettes de doses actives de différents progestatifs proposées dans 3 brevets différents (tableau 1).
   On constate que la limite inférieure pour les différents progestatifs varie dans un rapport de 2,4 à 50 tandis que la dose supérieure varie dans un rapport de 1 à 50. Donc, pour des indications de même type, les fourchettes de doses varient d'un brevet à l'autre d'une manière considérable, ce qui montre que le système d'équivalence n'apporte pas de crédibilité aux relations qui pourraient être établies entre progestatifs.
2) Au-delà de cela, on peut légitimement penser que les doses revendiquées devraient être basées sur des données de pharmacologie clinique et / ou de clinique préalablement publiées et communément admises. Or, si on considère les doses définies comme dans les brevets PLUNKETT, on voit aisément que, dans la plupart des cas, les doses actives publiées il y a déjà longtemps, c'est-à-dire avant que ces brevets ne soient déposés (NEUMANN, 1977) ou à une date plus récente (KUHL, 1996), sont très cohérentes mais ne sont que rarement comprises dans les fourchettes de doses revendiquées dans les brevets au nom de PLUNKETT (tableau 2).
Cette constatation est également valide si on prend en compte, au lieu des doses actives comme précédemment, le rapport entre les doses actives en prenant comme référence (=1) le norgestrel (tableau 3).

**Tableau n° 1**

| **Doses (µg/j) des différents progestatifs revendiqués selon les brevets** | | | |
|---|---|---|---|
| **PROGESTATIF** | **BREVET** | **DOSE (µg/j)** | |
| | | **Mini** | **Maxi** |
| Levonorgestrel | WO 95/17194 | 60 | 125 |
| Levonorgestrel | EP 025607 A1 | 25 | 100 |
| Levonorgestrel | PLUNKETT | 25 | 75 |
| | | | |
| Gestodene | WO 95/17194 | 50 | 75 |
| Gestodene | EP 025607 A1 | 10 | 70 |
| | | | |
| Desogestrel | WO 95/17194 | 60 | 150 |
| Desogestrel | EP 025607 A1 | 25 | 100 |
| | | | |
| 3 ketodesogestrel | WO 95/17194 | 60 | 150 |
| 3 ketodesogestrel | EP 025607 A1 | 25 | 100 |
| | | | |
| Noresthisterone | WO 95/17194 | 350 | 750 |
| Noresthisterone | EP 025607 A1 | 85 | 350 |
| Noresthisterone | PLUNKETT | 150 | 1000 |
| | | | |
| Norethisterone Acét | PLUNKETT | 100 | 1000 |
| | | | |
| Norgestimate | WO 95/17194 | 200 | 300 |
| | | | |
| Norgestrel | PLUNKETT | 50 | 150 |
| | | | |
| Ethynodiol diacétate | PLUNKETT | 100 | 1000 |
| Dihydrogesterone | PLUNKETT | 5000 | 30000 |
| MPA | PLUNKETT | 1000 | 15000 |
| Norethynodrel | PLUNKETT | 200 | 5000 |
| Allylestrenol | PLUNKETT | 1000 | 10000 |
| Lynestrenol | PLUNKETT | 100 | 2000 |
| Quingestanol Acétate | PLUNKETT | 50 | 1000 |
| Medrogestone | PLUNKETT | 1000 | 10000 |
| Norgestriénone | PLUNKETT | 20 | 200 |
| Dimethistérone | PLUNKETT | 500 | 15000 |
| Ethistérone | PLUNKETT | 1000 | 25000 |
| | | | |
| Ciprotérone Acétate | PLUNKETT | 100 | 10000 |
| Ciprotérone Acétate | WO 95/17194 | 100 | 200 |

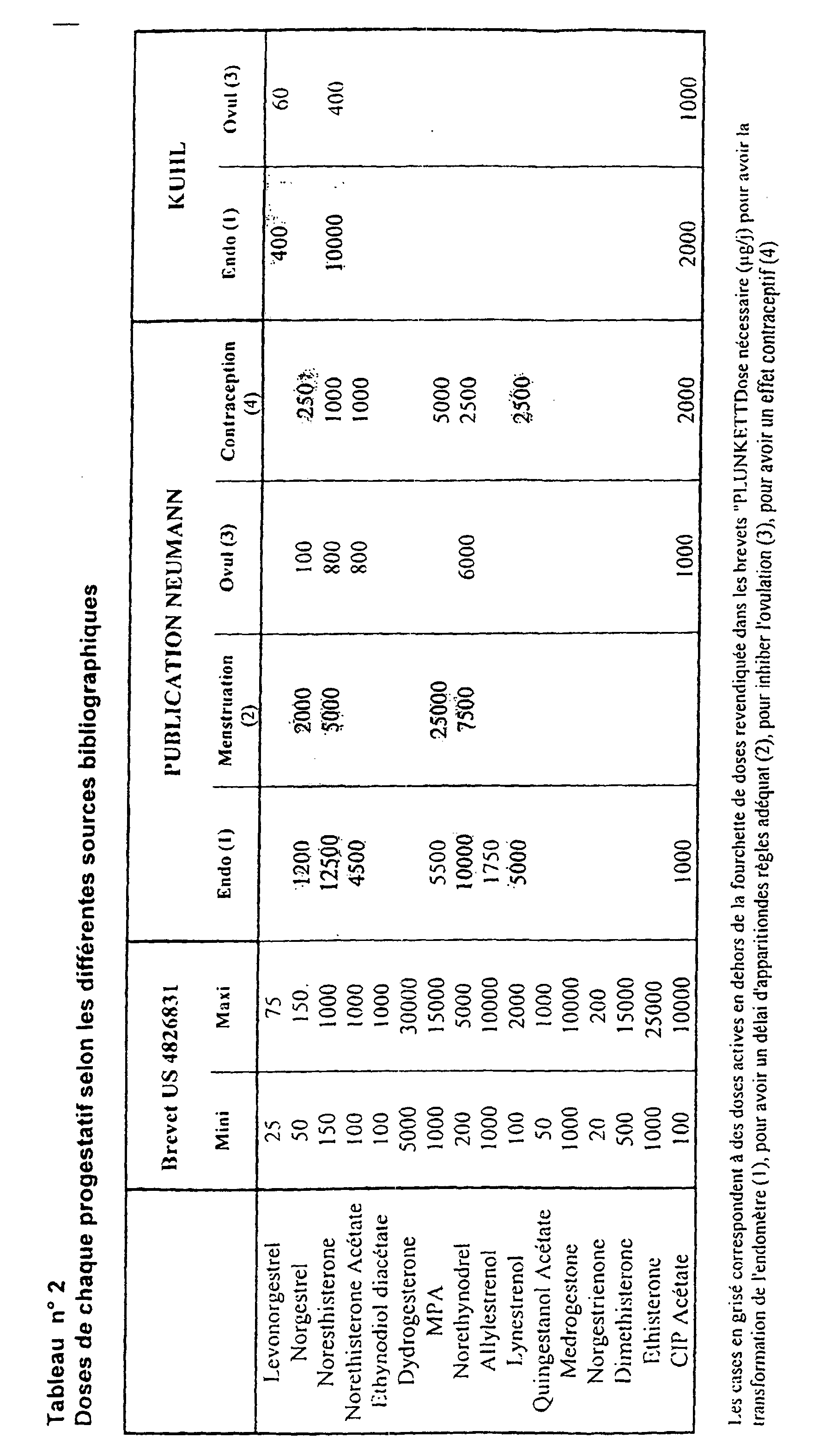

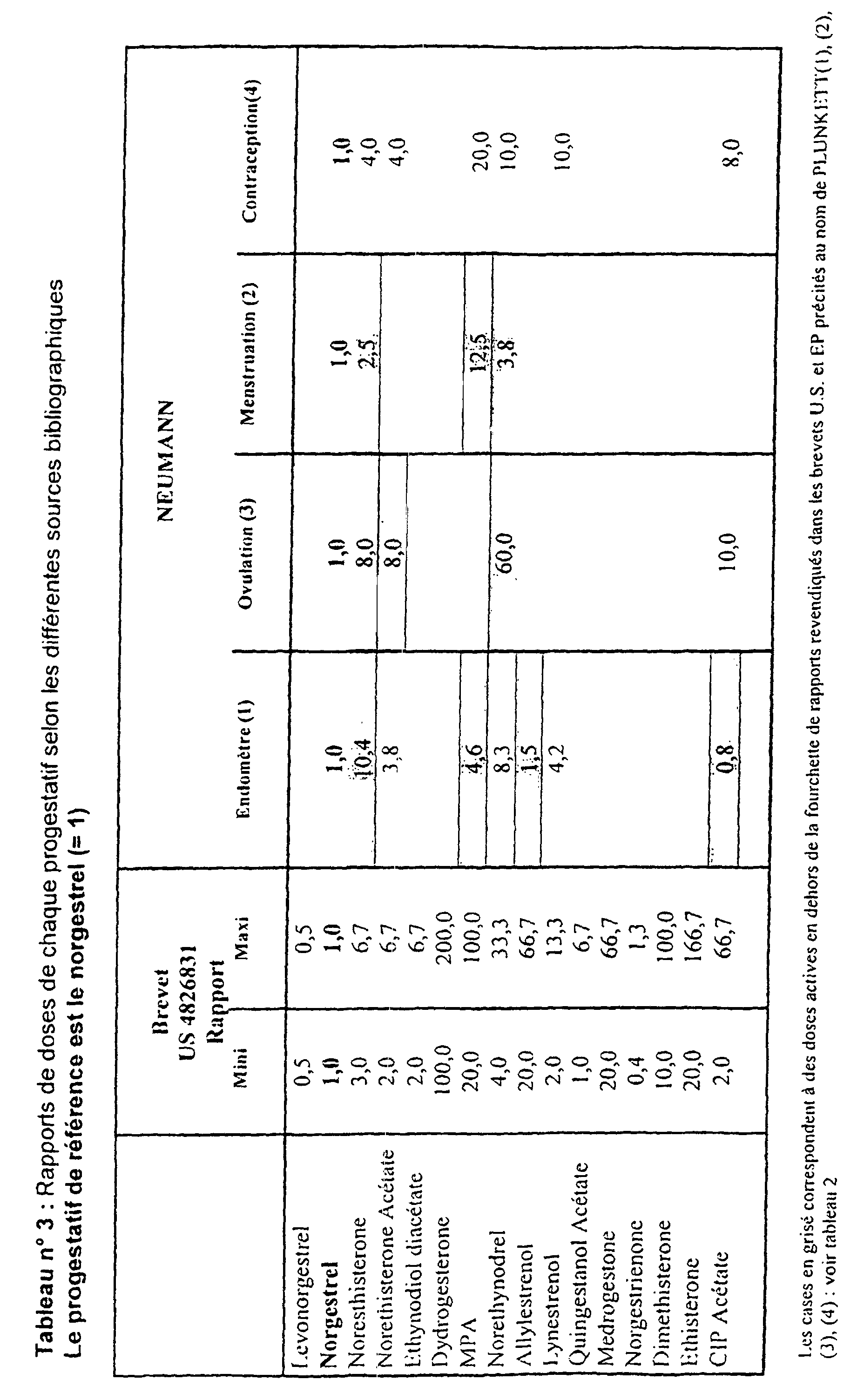

### Raisons liées aux revendications

1) Dans le brevet US précité, les revendications 1 et 2 sont consacrées au traitement continu; les seuls progestatifs revendiqués sont le dl-norgestrel et le levonorgestrel. Les revendications suivantes s'adressent au traitement discontinu pluriséquentiel, c'est-à-dire à un schéma thérapeutique différent de celui proposé dans la présente demande de brevet. Pour ce dernier type de régime thérapeutique, les progestatifs revendiqués sont plus nombreux, mais la liste en est précise et limitée comme il ressort de la rédaction du type Markush desdites revendications et elle n'inclut pas le nomegestrol et ses esters.
2) Le brevet européen ne revendique que le traitement combiné continu; les estrogènes et les progestatifs revendiqués sont listés dans des tableaux présents dans le corps du texte et rappelés dans les revendications. Là encore le nomegestrol et ses esters ne figurent pas dans les listes de progestatif pouvant être utilisés. Or l'acétate de nomegestrol se caractérise par un effet progestatif puissant, une absence d'effets androgéniques et estrogéniques résiduels ainsi que par un pouvoir anti-estrogénique puissant qui se traduit au niveau de l'endomètre par une forte activité anti-mitotique et, en conséquence, un fort effet atrophiant. De ce fait, il ne peut pas être assimilé aux autres progestatifs et concevoir une correspondance de doses par rapport à un autre progestatif pris comme référence, ne peut qu'être erronée. De plus, l'acétate de nomegestrol se caractérise par une très bonne tolérance; il n'a aucune action sur le profil des lipides, la tolérance aux glucides, la pression artérielle et les facteurs de coagulation même lorsqu'il est utilisé à des doses plus fortes que celles décrites dans la présente demande de brevet et pour des traitements de longue durée (BASDEVANT et al 1997). Cet aspect est très important car l'objectif qui consiste à utiliser le traitement substitutif le moins toxique possible en employant les doses les plus faibles possibles, est commun à tous les thérapeutes. En cela, l'acétate de nomegestrol se distingue de nombreux dérivés de 19-nortestostérone cités dans les brevets PLUNKETT, qui sont des progestatifs porteurs d'effets androgéniques et estrogéniques pouvant avoir des conséquences au niveau de l'endomètre, et qui ont aussi des effets métaboliques néfastes.
Pour les mêmes raisons que celles évoquées ci-dessus, aucune des nombreuses publications consacrées au traitement combiné continu de la ménopause ne peut affecter la présente invention puisqu' aucune de ces publications ne traite d'une association d'acétate de nomegestrol avec un estrogène. C'est par exemple le cas d'associations d'estradiol et d'acétate de norethistérone (STADBERG et al, 1996; DOREN et SCHNEIDER, 1996; DOREN et al, 1997; EIKEN et al, 1997; PIEGSA et al, 1997; HART et al, 1998), d'estradiol et de médrogestone (AFFINITO et al, 1998), d'estradiol et de norgestrel (WOLFE et PLUNKETT, 1994), de valérianate d'estradiol et d'acétate de chlormadinone (RAUCH et TAUBERT, 1993), d'estrogènes conjugués équins avec l'acétate de medroxyprogestérone (REUBINOFF et al, 1995; WOLFE et HUFF, 1995; MlZUNUMA et al, 1997) ou la medrogestone (RECKER et al, 1999).

### b) le fait que l'association estroprogestative se différencie de l'association précédemment brevetée par la Société Demanderesse

En effet, le brevet français 2.754.179 au nom de la Société Demanderesse revendiquait une association d'estradiol et d' acétate de nomegestrol pour le traitement substitutif combiné de la ménopause. La fourchette de doses revendiquée sur la base de l'expérience antérieure de l'acétate de nomegestrol dans le traitement séquentiel, allait de 1,5 à 3,75 mg, et se situait de préférence à 2,5 mg. Or les essais cliniques de plus grande échelle ont montré que, de façon inattendue, des doses très inférieures d'acétate de nomegestrol, et notamment de 0,325 à 1,25 mg, étaient capables d'assurer l'atrophie endométriale et un très bon contrôle des saignements. Cette observation est importante puisqu'elle permet de diminuer davantage les doses d'acétate de nomegestrol et donc de pouvoir l'utiliser avec une sécurité d'emploi encore accrue.
Les doses d'estradiol revendiquées dans ce brevet s'échelonnaient de 0,5 à 3 mg. Les mêmes doses d'estradiol sont utilisées ici, mais le rapport Estrogène / progestatif se trouve sensiblement modifié : 1 :5 au lieu de 6 : 1.

### c) la méthode de fabrication des formes pharmaceutiques adéquates

Selon l'invention, la méthode de fabrication permet de réunir dans une même forme pharmaceutique les deux principes actifs.

Les compositions selon l'invention, à base d'acétate de nomégestrol et d'estradiol libre ou estérifié, ou d'estrogènes conjugués équins sont administrés de façon continue ou intermittente (de 21 à 28 jours par mois).
Selon un mode d'exécution particulier de l'invention, les compositions contiennent une quantité d'acétate de nomegestrol s'échelonnant de 0,3 à 1,25 mg et une quantité d'estradiol libre ou estérifié, ou d'estrogènes conjugués équins s'échelonnant de 0,3 à 3 mg. De préférence, les formulations optimales contiennent de 0,625 à 1,25 mg d'acétate de nornegestrol associé à 0,5 à 1,5 mg d'estradiol libre ou 1,5 à 2 mg d'ester d'estradiol ou 0,312 à 0,625 mg d'estrogènes conjugués équins, par prise journalière.

Ce mode d'administration combiné est indiqué chez les femmes ménopausées, naturellement ou chirurgicalement ; la combinaison estroprogestative est destinée à compenser les troubles fonctionnels entraînés par l'hypoestrogénie de la ménopause, tout en maintenant une atrophie de l'endomètre et en évitant chez une majorité d'entre elles l'apparition d'hémorragie de privation.

Selon l'invention, les compositions pharmaceutiques sont obtenues par un procédé qui consiste à mélanger les principes actifs : acétate de nomegestrol et estradiol libre ou estérifié, ou des estrogènes conjugués équins avec un ou des excipients ou véhicules inertes, non toxiques, pharmaceutiquement acceptables.

Parmi les excipients on pourra citer les agents liants et solubilisants, les agents de compression, les agents de désintégration et les agents de glissement.
On peut soumettre ce mélange à une compression directe ou en plusieurs étapes pour former des comprimés que l'on peut protéger en surface, si désiré, par pelliculage, enrobage ou dragéification. La production de comprimés par compression directe permet de réduire au maximum la proportion d'agents de dilution, d'agents liants, d'agents de désintégration et d'agents de glissement.
La production de gélules pourra se faire en mélangeant les principes actifs à un diluant inerte et à un agent de glissement.

Les comprimés renferment, en particulier, des agents de dilution de la masse comme le lactose, le sorbitol pour compression directe, commercialisé sous la dénomination NEOSORB 60, le Palatinite qui est la marque déposée pour désigner un mélange équimolaire d'isomère de [D]glucopyranosido 1,6-mannitol et de [D]glucopyranosido 1,6-glucitol cristallisé avec deux molécules d'eau, le mannitol, le sorbitol ou le mélange lactose/PVP vendu sous la dénomination Ludipress.
Les agents liants de compression sont en général des celluloses microcristallines comme celles vendues sous la dénomiination AVICEL PH 101 ou AVICEL PH 102.
La polyvinylpyrrolidone joue également un rôle important et facilite l'agglomération des poudres et la compressibilité de la masse. On utilise à cette fin des polyvinylpyrrolidones de poids moléculaire compris entre 10000 et 30000 comme la Povidone, le Kollidon de grade compris entre 12 et 30.
Le mélange contient également des agents de glissement ou anti-électrostatiques qui évitent que la poudre ne s'agglomère dans les trémies d'alimentation. On peut citer, à cet égard, les silices colloïdales vendues sous la dénomination AEROSIL 100 ou AEROSIL 200.
Le mélange contient aussi des agents de désintégration qui permettent une désintégration ou un délitement conforme aux normes pharmaceutiques. On pourra citer comme agents de désintégration utiles, les polymères de vinylpyrrolidones réticulées telles que celles vendues sous les dénominations Polyplasdone ou Polyclar AT, les carboxyméthylamidons comme ceux vendus sous les dénominations Amijel ou Explotab, les carboxyméthylcelluloses réticulées comme le composé vendu sous la dénomination AC-DI-SOL.
En outre, la préparation contient des agents de lubrification qui facilitent la compression et l'éjection du comprimé sur les machines à comprimer. On pourra citer comme agents de lubrification, le palmitostéarate de glycérol vendu sous la dénomination Précirol, le stéarate de magnésium, l'acide stéarique ou le Talc.
Après compression, les comprimés peuvent être enrobés pour assurer leur conservation ou faciliter leur déglutition.
Les agents d'enrobage sont soit cellulosiques comme le phtalate de cellulose (Sepifilm, Pharmacoat), soit polyvinyliques du type OPADRY PVA ou Sépifilm ECL, soit saccharosiques comme le sucre pour dragéification du type Sépisperse DR, AS, AP, OU K (colorés).

Les comprimés enrobés ou non, peuvent, en outre, être colorés en surface ou dans la masse, par des colorants minéraux, végétaux ou synthétiques (par ex. laque au jaune de quinoléine ou E 104 ou oxydes de fer).
Les proportions des différents constituants varient selon la nature du comprimé à réaliser.
Les agents de dilution varient de 20 à 75 % de la masse totale, les agents de glissement de 0,1 à 2 % de la masse totale, les agents liants de compression varient de 2 à 20 %, la polyvinylpyrrolidone de 0,5 à 15 %, les agents de désintégration varient de 2 à 5,5 % pour la polyvinylpyrrolidone réticulée ou pour le carboxyméthylamidon, de 2,0 à 3,0 % pour la croscarmellose.
Les quantités d'agents de lubrification varient en fonction de la nature de l'agent de 0,1 à 3,0 %.
Les compositions selon l'invention sont destinées à être administrées une fois par jour. Cependant, en fonction des besoins thérapeutiques, l'administration peut être fragmentée (deux fois par jour) ou bien au contraire, renouvelée (deux comprimés par jour).
Les exemples suivants illustrent l'invention.

### EXEMPLE I : exemples de formulations

L'association d'acétate de nomegestrol et d'estradiol est présentée sous forme de comprimés nus ou pelliculés.
On pourra soit soumettre le mélange des ingrédients à une compression directe, soit réaliser au préalable un prémélange d'estradiol puis y incorporer l'acétate de nomegestrol et le reste des excipients sous forme sèche. Le prémélange d'estradiol est fabriqué par dissolution de l'estradiol dans une solution alcoolique de cellulose microcristalline, PVP et lactose, puis séchage, broyage et calibrage. Ce procédé est avantageux car les comprimés fabriqués à partir d'un prémélange d'estradiol présentent un profil de dissolution de l'estradiol sensiblement amélioré par rapport à ceux qui sont fabriqués en compression directe.

Le mélange final pourra contenir de 1,5 à 5 % d'estradiol dans de la povidone (5 à 25%), de la cellulose microcristalline (5 à 15 %) et du lactose (qsp 100%). Il pourra être avantageux d'introduire un agent anti-oxydant tel que par exemple l'alpha-tocophérol ou l'acide ascorbique, lors de la fabrication du prémélange.

. On pourra citer comme exemple de prémélange :

| FORMULATIONS | en mg / 1 Comprimé | 1 en % |
|---|---|---|
| Estradiol | 1,50 | 1,82 |
| PVP K25 | 13,50 | 16,36 |
| Lactose 8195 | 60,00 | 72,73 |
| Cellulose microcristal | 7,50 | 9,09 |
| **TOTAL EN SEC** | 82,50 | **100,00** |

Ce prémélange est introduit dans le mélange final pour obtenir un comprimé par compression directe.
Les comprimés terminés, nus, pèsent en général de 60 à 200 mg et ont la formule globale suivante :

### FORMULATIONS DES COMPRIMES NUS

| **Composition** | |
|---|---|
| | **en mg/par comprimé** |
| - Estradiol (prémélange qsp) | **0,3 à 3,0** |
| - Acétate de nomegestrol | **0,300 à 1,250** |
| - Silice colloïdale | 0,400 à 2,000 |
| - Crospovidone | 2,500 à 4,000 |
| - Lactose | 60,000 à 80,000 |
| - Cellulose | 10,000 à 25,000 |
| - Acide stéarique | 0,900 à 3,00 |
| - Talc | 0,450 à 1,500 |

A titre d'exemple, on pourra citer les comprimés pesant 185 mg, de formule suivante :

| **Exemple de formulations (FU = formulation unitaire) comprimés de 185 mg** | | |
|---|---|---|
| **FORMULATIONS** | **FU mg/1 cp 185 mg** | **FU %** |
| Estradiol | 1,500 | 0,811 |
| Acétate de nomegestrol | **0,625** | 0,338 |
| Lactose | 131,790 | 71,238 |
| Cellulose (Avicel PH 101) | 27,810 | 15,032 |
| Povidone (K25) | 13,500 | 7,297 |
| Précirol AT05 | 2,780 | 1,503 |
| Silice colloïdale (Aérosil 200) | 1,000 | 0,540 |
| Crospovidone (Polyplasdone XL)² | 6,000 | 3,243 |
| **TOTAL** | **185,00** | **100,00** |

| **Exemple de formulations (FU = formulation unitaire) comprimés de 185 mg** | | |
|---|---|---|
| **FORMULATIONS** | **FU mg/1 cp 185 mg** | **FU %** |
| Estradiol | 0,500 | 0,270 |
| Acétate de nomegestrol | **0,625** | 0,339 |
| Lactose | 136,787 | 73,934 |
| Cellulose (Avicel PH 101) | 32,813 | 17,736 |
| Povidone (K25) | 4,500 | 2,432 |
| Précirol AT05 | 2,775 | 1,500 |
| Silice colloïdale (Aérosil 200) | 1,000 | 0,540 |
| Crospovidone (Polyplasdone XL) | 6,000 | 3,243 |
| **TOTAL** | **185,00** | **100,00** |

| **Exemple de formulations (FU = formulation unitaire) comprimés de 120 mg** | | |
|---|---|---|
| **FORMULATIONS** | **FU mg/1 cp 120 mg** | **FU %** |
| Estradiol | 1,500 | 1,250 |
| Acétate de nomegestrol | **1,250** | 1,042 |
| Lactose | 84,000 | 70,000 |
| Cellulose (Avicel PH 101) | 11,250 | 9,375 |
| Povidone (K25) | 13,500 | 11,250 |
| Silice colloïdale (Aérosil 200) | 1,000 | 0,833 |
| Crospovidone (Polyplasdone XL.) | 3,000 | 2,500 |
| Stéarate de magnésium | 1,000 | 0,833 |
| Talc | 1,000 | 0,833 |
| Acide stéarique AC/50VG | 2,500 | 2,083 |
| **TOTAL** | **120,00** | **100,00** |

| **Exemple de formulations (FU = formulation unitaire) comprimés de 120 mg** | | |
|---|---|---|
| **FORMULATIONS** | **FU mg/1 cp 120 mg** | **FU %** |
| Estradiol | 0,500 | 0,417 |
| Acétate de nomegestrol | **0,625** | 0,521 |
| Lactose | 89,000 | 74,167 |
| Cellulose (Avicel PH 101) | 16,875 | 14,062 |
| Povidone (K25) | 4,500 | 3,750 |
| Silice colloïdale (Aérosil 200) | 1,000 | 0,833 |
| Crospovidone (Polyplasdone XL) | 3,000 | 2,500 |
| Stéarate de magnésium (végétal) | 1,000 | 0,833 |
| Talc | 1,000 | 0,833 |
| Acide stéarique AC/50VG (végétal) | 2,500 | 2,083 |
| **TOTAL** | **120,00** | **100,00** |

| **Exemple de formulations (FU = formulation unitaire) comprimés de 80 mg** | | |
|---|---|---|
| **FORMULATIONS** | **FU mg/1 cp 80 mg** | **FU %** |
| Estradiol | 0,500 | 0,625 |
| Acétate de nomegestrol | **0,625** | 0,781 |
| Kollidon 25 | 4,500 | 5,625 |
| Lactose M | 59,735 | 74,669 |
| Cellulose (Avicel PH 101) | 12,000 | 15,000 |
| Crospovidone(Polyplasdone XL) | 0,800 | 1,000 |
| Talc | 0,700 | 0,875 |
| Silice colloïdale (Aérosil 200) | 0,440 | 0,550 |
| Stéarate de magnésium (végétal) | 0,700 | 0,875 |
| **TOTAL** | **80,0** | **100,00** |

| **Exemple de formulations (FU = formulation unitaire) comprimés de 80 mg** | | |
|---|---|---|
| **FORMULATIONS** | **FU mg/1 cp 80 mg** | **FU %** |
| Estradiol | 1,000 | 1,250 |
| Acétate de nomegestrol | **1,250** | **1,563** |
| Kollidon 25 | 9,000 | 11,250 |
| Lactose M | 54,110 | 67,637 |
| Cellulose (Avicel PH 101) | 12,000 | 15,000 |
| Crospovidone(Polyplasdone XL) | 0,800 | 1,000 |
| Talc | 0,700 | 0,875 |
| Silice colloïdale (Aérosil 200) | 0,440 | 0,550 |
| Stéarate de magnésium (végétal) | 0,700 | 0,875 |
| **TOTAL** | **80,00** | **100,00** |

Ces comprimés peuvent être pelliculés avec par exemple :
- des agents de pelliculage à base d'alcool polyvinylique de type OPADRY PVA "barrière humidité" (alcool polyvinylique, dioxyde de titane, talc purifié, lécithine, gomme xanthane, pigments, laques),
ou
- des agents de pelliculage à base de cellulose de type SEPIFILM L.P. [H.P.M.C. (hydroxypropylméthylcellulose)], cellulose microcristalline, acide stéarique, pigments, laques).

### Exemple II

### Dissociation des effets antimitotiques et différenciant de l'acétate de nomegestrol sur la cellule endométriale

Des femmes ménopausées, naturellement ou chirurgicalement, ont été soumises à un traitement estroprogestatif séquentiel. Elles ont reçu de J1 à J12 , 30 µg d'éthinyl estradiol puis du 13^{ème} au 22^{éme} jour la même dose d'éthinyl estradiol associée à différentes doses d'acétate de nomégestrol ou d'acétate de chlormadinone. Les doses étaient les suivantes :

| | | | | | | | | |
|---|---|---|---|---|---|---|---|---|
| acétate de nomegestrol en mg : | 0,1 | 0,25 | 0,5 | 1 | | 2,5 | 5 | et 10 |
| acétate de chlormadinone en mg : | 0,1 | | 0,5 | 1 | 2 | | 5 | et 10 |

Le nombre de femmes était compris entre 2 et 11 selon les groupes.
Il y avait ensuite une pose thérapeutique de 7 jours, suivie par un 2^{ème} cycle traité.

Les paramètres pris en compte ont été :
- le délai de survenue des règles après l'arrêt du traitement après le 1^{er} et le 2^{ème} cycle de traitement ;
- l'aspect histologique de l'endomètre récupéré lors d'une biopsie réalisée entre le 17^{ème} et le 20^{ème} jour du second cycle.

Les résultats montrent que :
- le délai d'apparition des règles est fonction de la dose et similaire pour les 2 produits. La dose nécessaire pour que les règles n'apparaissent pas avant la fin du traitement est comprise entre 0,2 et 0,3 mg/j pour les 2 produits (tab 1);
- la transformation de l'endomètre en endomètre sécrétoire est totale avec les 2 produits à partir de 1 mg/j ; elle diminue pour les doses les plus élevées (10 mg/j) ;
- l'activité de prolifération, exprimée par le nombre de mitoses dans les cellules glandulaires apparaît plus fortement inhibée par l'acétate de nomegestrol que par l'acétate de chlormadinone. Il n'y a plus de mitose chez les femmes traitées avec une dose égale ou supérieure à 0,5 mg/j d'acétate de nomegestrol alors que les mitoses sont encore présentes avec une dose quotidienne de 1 mg/j d'acétate de chlormadinone (tab 2).

On peut donc conclure qu'au niveau endométrial, l'acétate de nomegestrol et l'acétate de chlormadinone ne sont pas comparables : l'activité de transformation sécrétoire est comparable mais l'acétate de nomegestrol se caractérise par une très forte activité antimitotique et antiprolifératrive.

**Tableau 1 :**

| **Délai d'apparition des règles (jours) après l'arrêt du traitement** | | | | | | | | |
|---|---|---|---|---|---|---|---|---|
| **Progestatif** | **Dose (mg/j)** | | | | | | | |
| | 0,1 | 0,25 | 0,5 | 1,0 | 2,0 | 2,5 | 5 | 10 |
| Acétate de nomegestrol | - 0,5 ± 1,5 | - 0,5 ±0,5 | 1,5 ± 0,5 | 2,7 ± 0,2 | | 3,2 ± 0,2 | 4,3 ± 0,4 | 3,8 ± 0,8 |
| Acétate de Chlormadinone | - 2,3 ± 0,5 | | 2,0 ± 0,6 | 3,0 | 3,8 ± 0,3 | | 5,5 ± 1,5 | 5,5 ± 0,5 |

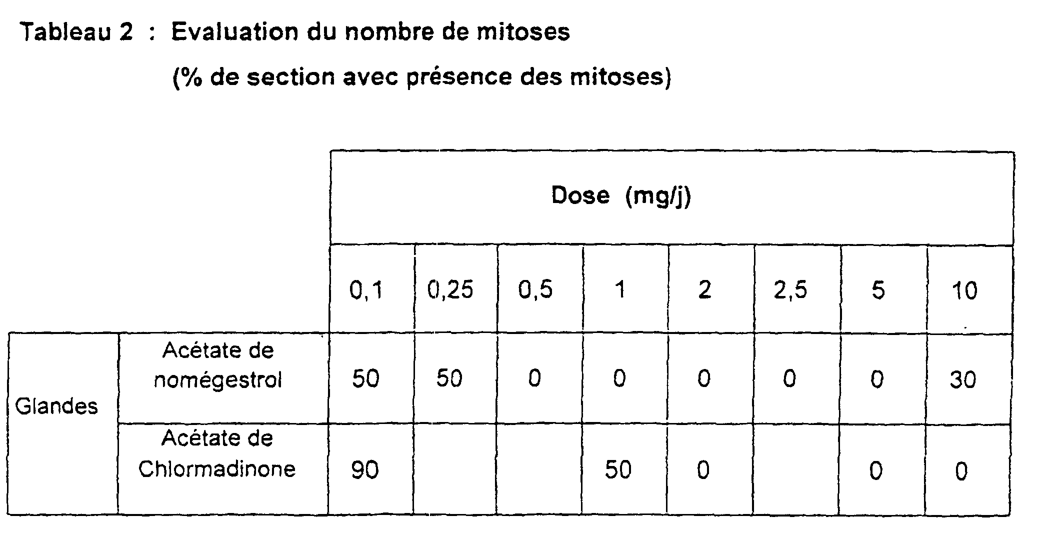

### EXEMPLE III

Une étude a été conduite pour tester les effets sur l'endomètre de l'association combinée continue d'une dose d'estradiol oral, équivalente à 1,5 mg et de plusieurs doses d'acétate de nomegestrol.

Elle a consisté à traiter pendant 6 mois consécutifs 179 femmes ménopausées depuis au moins 3 ans, par 1,5 mg par jour d'estradiol combinés en continu avec 4 doses différentes d'acétate de nomegestrol : 5 mg/jour (n=47) ; 2,5 mg/jour (n=42) ; 1,25 mg/jour (n=43) et 0,625 mg/jour (n=47).

L'impact de ces 4 associations sur l'endomètre a été évalué en recueillant les caractéristiques des saignements génitaux, en mesurant l'épaisseur de l'endomètre par échographie endovaginale avant et en fin de traitement et en réalisant une biopsie de l'endomètre avant et en fin de traitement.

Le pourcentage des femmes n'ayant présenté aucun saignement génital durant la totalité du traitement a été respectivement de 42,5 - 58,1 - 52,4 et 68,1 %, avec les doses de 0,625 - 1,25 - 2,5 et 5 mg d'acétate de nomegestrol par jour. Les pourcentages observés ne sont pas statistiquement différents entre groupes, mais la relation entre dose et incidence des saignements est significative.

Les tableaux ci-joints indiquent pour chaque dose d'acétate de nomegestrol les résultats de l'examen échographique et de la biopsie de l'endomètre réalisés à la fin des 6 mois de traitement.

En fin de traitement, l'épaisseur moyenne de l'endomètre n'est pas différente entre les groupes. L'augmentation de l'épaisseur endométriale sous traitement est de 0,39 mm en moyenne avec la plus faible dose d'acétate de nomegestrol. Cet accroissement augmente légèrement avec la dose, pour atteindre 1,56 mm dans le groupe des femmes recevant 5 mg/jour de progestatif, mais la relation entre variation de l'épaisseur et variation de dose n'atteint pas le seuil de la signification statistique.

Les biopsies de l'endomètre examinées en fin d'étude n'ont révélé aucun aspect prolifératif ou hyperplasique de la muqueuse utérine. Le plus fort pourcentage d'endomètres secrétoires a été observé chez les femmes ayant reçu la plus forte dose de progestatif ; il diminue de façon progressive et statistiquement significative avec la dose. Au contraire, le plus fort pourcentage d'endomètres atrophiques a été constaté avec la plus faible dose de progestatif et il baisse avec la dose.

Ces résultats sont inattendus dans la mesure où ils montrent que de faibles doses d'acétate de nomegestrol administrées de façon combinée continue avec un estrogène sont capables d'empêcher la croissance de la muqueuse utérine et de la maintenir dans un état atrophique alors que, contrairement aux doses plus fortes, elles sont insuffisantes pour engendrer une transformation secrétoire de l'endomètre.

Cette étude met ainsi en évidence un découplage surprenant de l'effet anti-estrogène et de l'effet progestatif de l'acétate de nomegestrol, administré de façon combinée continue avec les estrogènes.

L'effet anti-estrogène est prépondérant puisqu'il est décelable lorsque le progestatif, administré en continu avec un estrogène, est donné à des doses faibles. Ces doses sont insuffisantes pour entraîner les transformations secrétoires de la muqueuse utérine. A plus fortes doses et avec le même schéma thérapeutique, l'effet secrétoire prédomine, sans toutefois permettre une prolifération excessive de l'endomètre.

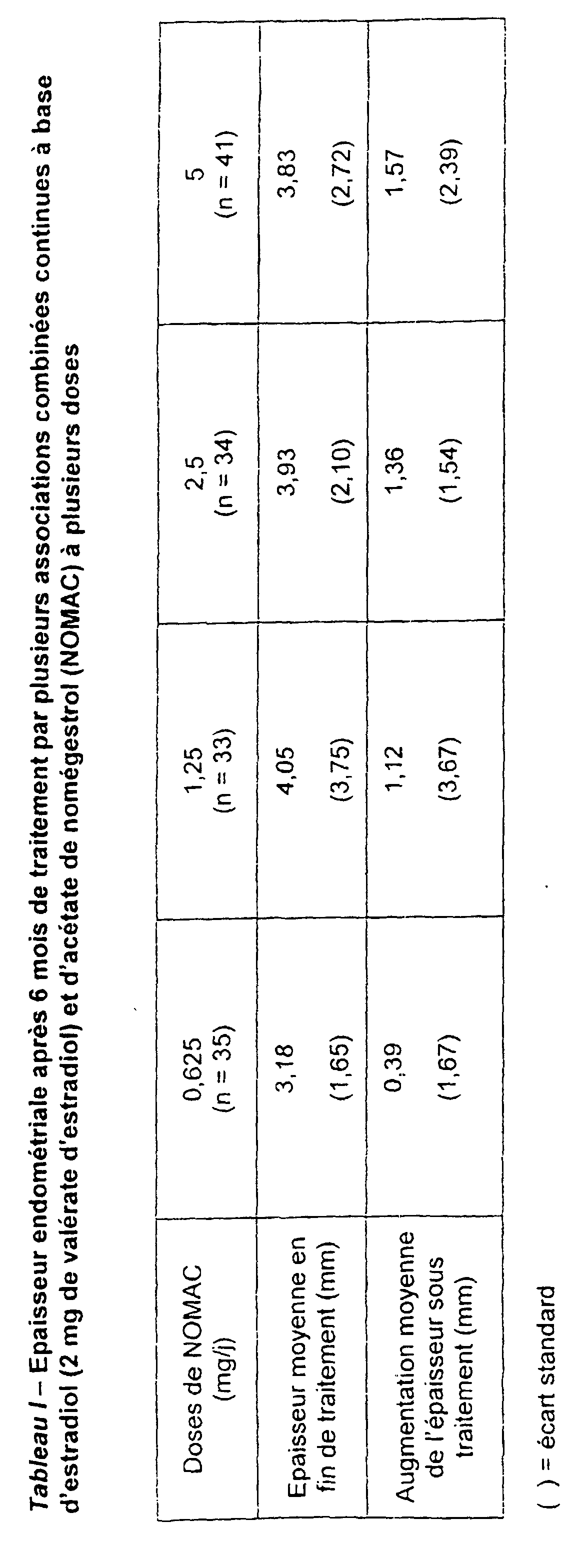

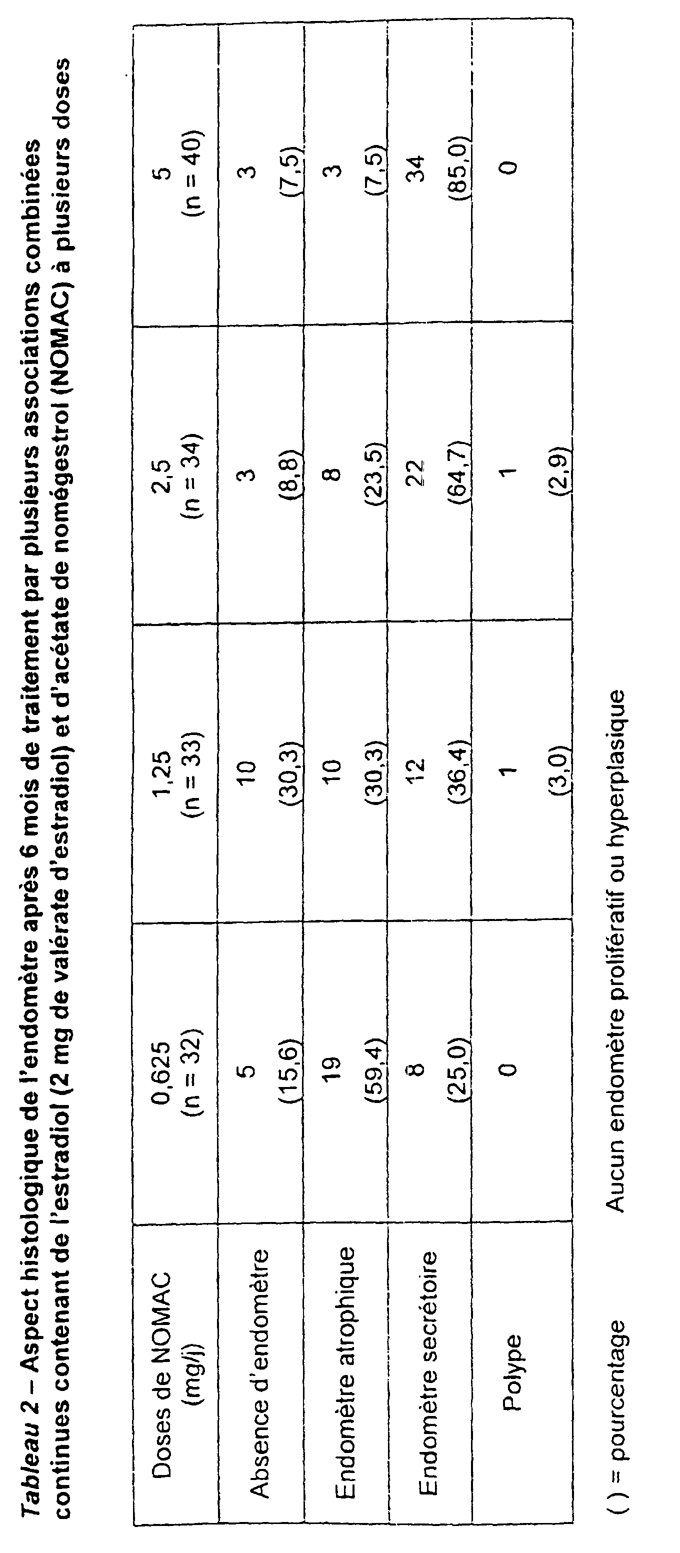

## Revendications

1. Utilisation d'une association estroprogestative combinée pour l'administration par voie orale en vue de la préparation d'un médicament destiné à la correction des carences estrogéniques chez la femme et plus spécialement chez la femme ménopausée, dans laquelle l'association contient un estrogène à une dose s'échelonnant de 0,3 à 3 mg, et un progestatif choisi parmi le nomegestrol ou l'un de ses esters à une dose s'échelonnant de 0,3 à 1,25 mg, en association ou en mélange avec un ou plusieurs excipients non toxiques, inertes, pharmaceutiquement acceptables.

2. Utilisation d'une association estroprogestative combinée pour l'administration par voie orale en vue de la préparation d'un médicament destiné à prévenir l'ostéoporose et les maladies cardiovasculaires chez la femme ménopausée, dans laquelle l'association contient un estrogène à une dose s'échelonnant de 0,3 à 3 mg, et un progestatif choisi parmi le nomegestrol ou l'un de ses esters à une dose s'échelonnant de 0,3 à 1,25 mg, en association ou en mélange avec un ou plusieurs excipients non toxiques, inertes, pharmaceutiquement acceptables.

3. Utilisation selon la revendication 1 ou 2, dans laquelle l'estrogène est choisi parmi le 17β-estradiol, libre ou estérifié, ou des estrogènes conjugués équins.

4. Utilisation selon l'une des revendications 1 à 3, dans laquelle l'estrogène est le 17β-estradiol.

5. Utilisation selon l'une des revendications 1 à 3, dans laquelle l'estrogène est un ester d'estradiol.

6. Utilisation selon la revendication 5, dans laquelle l'estrogène est le valérate d'estradiol.

7. Utilisation selon l'une des revendications 1 à 3, dans laquelle l'estrogène est constitué par des estrogènes conjugués équins.

8. Utilisation selon la revendication 4, dans laquelle l'estradiol libre est présent à une dose de 0,5 à 1,5 mg par prise unitaire.

9. Utilisation selon la revendication 5 ou 6, dans laquelle l'ester d'estradiol est présent à une dose de 1,5 à 2 mg par prise unitaire.

10. Utilisation selon la revendication 7, dans laquelle les estrogènes conjugués équins sont présents à une dose de 0,312 à 0,625 mg par prise unitaire.

11. Utilisation selon l'une des revendications 1 à 10, dans laquelle le progestatif est l'acétate de nomegestrol.

12. Utilisation selon la revendication 11, dans laquelle l'acétate de nomegestrol est présent à une dose allant de 0,625 à 1,25 mg par prise unitaire.

13. Utilisation selon l'une des revendications 1 à 12, en vue de la production d'un médicament destiné à être administré de façon continue.

14. Utilisation selon l'une des revendications 1 à 12, en vue de la production d'un médicament destiné à être administré de façon intermittente.

## Claims

1. Use of a combined estroprogestative association to be administered by the oral route for the preparation of a medicament intended to correct estrogen deficiencies in women, more particularly in menopausal women, wherein the association contains an estrogen at a dose ranging from 0.3 to 3 mg, and a progestative selected from nomegestrol and esters thereof at a dose ranging from 0.3 to 1.25 mg, in combination or admixture with one or more non-toxic, inert, pharmaceutically acceptable excipient(s).

2. Use of a combined estroprogestative association to be administered by the oral route for the preparation of a medicament intended to prevent osteoporosis and cardiovascular diseases in menopausal women, wherein the association contains an estrogen at a dose ranging from 0.3 to 3 mg, and a progestative selected from nomegestrol and esters thereof at a dose ranging from 0.3 to 1.25 mg, in combination or admixture with one or more non-toxic, inert, pharmaceutically acceptable excipient(s).

3. Use according to claim 1 or 2, wherein the estrogen is selected from free or esterified 17β-estradiol and conjugated equine estrogens.

4. Use according to one of claims 1 to 3, wherein the estrogen is 17β-estradiol.

5. Use according to one of claims 1 to 3, wherein the estrogen is an ester of estradiol.

6. Use according to claim 5, wherein the estrogen is estradiol valerate.

7. Use according to one of claims 1 to 3, wherein the estrogen consists of conjugated equine estrogens.

8. Use according to claim 4, wherein the free estradiol is present in an amount of from 0.5 to 1.5 mg by unit dose.

9. Use according to claim 5 or 6, wherein the ester of estradiol is present in an amount of from 1.5 to 2 mg by unit dose.

10. Use according to claim 7, wherein the conjugated equine estrogens are present in an amount of from 0.312 to 0.625 mg by unit dose.

11. Use according to one of claims 1 to 10, wherein the progestative is nomegestrol acetate.

12. Use according to claim 11, wherein nomegestrol acetate is present in an amount of from 0.625 to 1.25 mg by unit dose.

13. Use according to one of claims 1 to 12, for the preparation of a medicament intended to be administered continuously.

14. Use according to one of claims 1 to 12, for the preparation of a medicament intended to be administered intermittently.

## Patentansprüche

1. Verwendung einer kombinierten östro-progestativen Assoziation zur oralen Verabreichung zur Herstellung eines Medikaments, das zur Korrektur von Östrogenmängeln bei der Frau und insbesondere bei der Frau in der Menopause bestimmt ist, wobei die Assoziation ein Östrogen in einer Dosis im Bereich von 0,3 bis 3 mg und ein Progestativum, ausgewählt aus Nomegestrol oder einem seiner Ester, in einer Dosis im Bereich von 0,3 bis 1,25 mg in Assoziation oder im Gemisch mit einem oder mehreren nicht toxischen, inerten, pharmazeutisch verträglichen Exzipientien enthält.

2. Verwendung einer kombinierten östro-progestativen Assoziation zur oralen Verabreichung zur Herstellung eines Medikaments, das zur Prävention von Osteoporose und kardiovaskulären Erkrankungen bei der Frau in der Menopause bestimmt ist, wobei die Assoziation ein Östrogen in einer Dosis im Bereich von 0,3 bis 3 mg und ein Progestativum, ausgewählt aus Nomegestrol oder einem seiner Ester, in einer Dosis im Bereich von 0,3 bis 1,25 mg in Assoziation oder im Gemisch mit einem oder mehreren nicht toxischen, inerten, pharmazeutisch verträglichen Exzipientien enthält.

3. Verwendung nach Anspruch 1 oder 2, wobei das Östrogen aus freiem oder verestertem 17ß-Östradiol oder aus equinen, konjugierten Östrogenen ausgewählt ist.

4. Verwendung nach einem der Ansprüche 1 bis 3, wobei das Östrogen 17ß-Östradiol ist.

5. Verwendung nach einem der Ansprüche 1 bis 3, wobei das Östrogen ein Östradiolester ist.

6. Verwendung nach Anspruch 5, wobei das Östrogen Östradiolvalerat ist.

7. Verwendung nach einem der Ansprüche 1 bis 3, wobei das Östrogen aus equinen, konjugierten Östrogenen besteht.

8. Verwendung nach Anspruch 4, wobei das freie Östradiol in einer Dosis von 0,5 bis 1,5 mg pro Dosierungseinheit vorhanden ist.

9. Verwendung nach Anspruch 5 oder 6, wobei der Östradiolester in einer Dosis von 1,5 bis 2 mg pro Dosierungseinheit vorhanden ist.

10. Verwendung nach Anspruch 7, wobei die equinen, konjugierten Östrogene in einer Dosis von 0,312 bis 0,625 mg pro Dosierungseinheit vorhanden sind.

11. Verwendung nach einem der Ansprüche 1 bis 10, wobei das Progestativum Nomegestrolacetat ist.

12. Verwendung nach Anspruch 11, wobei das Nomegestrolacetat in einer Dosis von 0,625 bis 1,25 mg pro Dosierungseinheit vorhanden ist.

13. Verwendung nach einem der Ansprüche 1 bis 12 zur Herstellung eines Medikaments, das zur kontinuierlichen Verabreichung bestimmt ist.

14. Verwendung nach einem der Ansprüche 1 bis 12 zur Herstellung eines Medikaments, das zur diskontinuierlichen Verabreichung bestimmt ist.
